# EUROPEAN PATENT SPECIFICATION

(11) **EP 4 179 499 B1**
(45) Date of publication and mention of the grant of the patent: **01.07.2026**
(21) Application number: 21838666.2
(22) Date of filing: 09.07.2021
(51) Int. Cl.: G06T 7/00, G06V 20/69

(54) **PARAMETRIC MODELING AND INFERENCE OF DIAGNOSTICALLY RELEVANT HISTOLOGICAL PATTERNS IN DIGITIZED TISSUE IMAGES**
PARAMETRISCHE MODELLIERUNG UND INFERENZ VON DIAGNOSTISCH RELEVANTEN HISTOLOGISCHEN MUSTERN IN DIGITALISIERTEN GEWEBEBILDERN
MODÉLISATION PARAMÉTRIQUE ET INFÉRENCE DE MOTIFS HISTOLOGIQUES PERTINENTS POUR LE DIAGNOSTIC DANS DES IMAGES DE TISSU NUMÉRISÉES

(30) Priority: 09.07.2020 US 202063049690 P
(43) Date of publication of application: 17.05.2023
(73) Proprietor: University of Pittsburgh - of the Commonwealth System of Higher Education, Pittsburgh, Pennsylvania 15260 (US)
(72) Inventor: CHENNUBHOTLA, Srinivas C., Pittsburgh, Pennsylvania 15217 (US); TOSUN, Akif Burak, Pittsburgh, Pennsylvania 15237 (US); FINE, Jeffrey, Pittsburgh, Pennsylvania 15237 (US)
(74) Representative: Mewburn Ellis LLP
(86) International application number: PCT/US2021/041037
(87) International publication number: WO 2022/011226

(56) References cited:
- WO-A1-2016/075096
- US-A1- 2012 106 821
- US-A1- 2012 106 821
- US-A1- 2019 080 795
- US-A1- 2020 123 618
- TOSUN AKIF BURAK ET AL: "European Conference on Computer Vision", vol. 10434, 10 September 2017 (2017-09-10), Copenhagen, Denmark, pages 144 - 152, XP093178214, ISSN: 0302-9743, Retrieved from the Internet <URL:https://link.springer.com/content/pdf/10.1007/978-3-319-66185-8.pdf> DOI: 10.1007/978-3-319-66185-8 17
- DONG FEI ET AL: "Computational Pathology to Discriminate Benign from Malignant Intraductal Proliferations of the Breast", PLOS ONE, vol. 9, no. 12, 9 December 2014 (2014-12-09), US, pages e114885, XP093178285, ISSN: 1932-6203, DOI: 10.1371/journal.pone.0114885
- AKIF BURAK TOSUN ET AL: "Graph Run-Length Matrices for Histopathological Image Segmentation", IEEE TRANSACTIONS ON MEDICAL IMAGING, IEEE, USA, vol. 30, no. 3, 1 January 2011 (2011-01-01), pages 721 - 732, XP011349063, ISSN: 0278-0062, DOI: 10.1109/TMI.2010.2094200
- TOSUN AKIF BURAK ET AL: "HistoMapr: An Explainable AI (xAI) Platform for Computational Pathology Solutions", 24 June 2020, SPRINGER, PAGE(S) 204 - 227, XP047552352
- PARVATIKAR AKASH ET AL: "Medical Image Computing and Computer Assisted Intervention - MICCAI 2020 : 23rd International Conference, Lima, Peru, October 4-8, 2020, Proceedings, Part V", vol. 12265, 29 September 2020 (2020-09-29), Cham, pages 550 - 560, XP093178036, ISSN: 0302-9743, ISBN: 978-3-030-59722-1, Retrieved from the Internet <URL:https://link.springer.com/content/pdf/10.1007/978-3-030-59722-1.pdf> DOI: https://doi.org/10.1007/978-3-030-59722-1_53
- WIRTH, M.: "Shape Analysis & Measurement", UNIVERSITY OF GEORGIA, 2004, pages 1 - 97, XP055893737, Retrieved from the Internet <URL:http://www.cyto.purdue.edu/cdroms/micro2/content/education/wirth10.pdf>
- WESTON DAVID J., ADAMS NIALL M., RUSSELL RICHARD A., STEPHENS DAVID A., FREEMONT PAUL S.: "Analysis of Spatial Point Patterns in Nuclear Biology", PLOS ONE, vol. 7, May 2012 (2012-05-01), pages 1 - 15, XP055893753, Retrieved from the Internet <URL:https://journals.plos.org/plosone/article?id=10.1371/journal.pone.0036841>
- ELHAM AZIZI; AMBROSE J CARR; GEORGE PLITAS; ANDREW E CORNISH; CATHERINE KONOPACKI; SANDHYA PRABHAKARAN; JUOZAS NAINYS; KENMIN WU; : "Single-cell Map of Diverse Immune Phenotypes in the Breast Tumor Microenvironment", HHS PUBLIC ACCESS, 23 August 2018 (2018-08-23), pages 1 - 67, XP055603830, Retrieved from the Internet <URL:https://www.ncbi.nlm.nih.gov/pmc/articles/PMC6348010/pdf/nihms-977868.pdf>
- GERTYCH ARKADIUSZ, ING NATHAN, MA ZHAOXUAN, FUCHS THOMAS J, SALMAN SADRI, MOHANTY SAMBIT, BHELE SANICA, VELÁSQUEZ-VACCA ADRIANA, A: "Machine learning approaches to analyze histological images of tissues from radical prostatectomies", HHS PUBLIC ACCESS, December 2015 (2015-12-01), pages 197 - 208, XP055893775, Retrieved from the Internet <URL:https://europepmc.org/backend/ptpmcrender.fcgi?accid=PMC5062020&blobtype=pdf>

## Description

### GOVERNMENT CONTRACT

This invention was made with government support under grant # CA204826 awarded by the National Institutes of Health (NIH). The government has certain rights in the invention.

### FIELD OF THE INVENTION

The disclosed concept relates generally to digital pathology, and in particular, to a system and method for parametrically modelling a dictionary of diagnostically relevant histological patterns and using the modeling scheme to quantify the existence of the patterns in digital pathology images in order to be able to classify the state of a disease in the digital pathology images.

### BACKGROUND OF THE INVENTION

Advances in digital imaging technologies and computational power have now paved the way for a major shift in pathology workflow based on artificial intelligence (AI). It is now feasible to rapidly image microscope slides at clinical volumes, and it is now permissible to use these digital pathology images for real clinical diagnosis given recent regulatory approvals. A critical addition is computational pathology in the form of novel machine learning (ML) tools that pathologists could use to greatly improve their diagnostic performance, especially in terms of accuracy and efficiency. Such tools could also be applied throughout the pathology laboratory for other applications such as case triage or automated real-time quality assurance. Numerous studies have now shown early promise, and it is becoming clear that pathologists and their patients could greatly benefit from access to powerful computational pathology tools.

There is widespread enthusiasm for AI in digital pathology, but this is strongly tempered by caution and reasonable concern about potential risks. Further, almost all early computational pathology attempts have used convolutional neural networks, also known as deep learning. Deep learning is powerful, but it is opaque, like a "black-box" that one cannot open to peer inside and see what it is doing or exactly how it is working, or even whether it is working as intended. Such systems give answers, but do not allow the pathologist to ask "why?". Tosun Akif Burak ET AL: "Histological Detection of High-Risk Benign Breast Lesions from Whole Slide Images" in European Conference on Computer Vision (10 September 2017, Springer Berlin Heidelberg, Copenhagen, Denmark) describes a computational pathology pipeline for histological diagnosis of high-risk benign breast lesions from whole slide images (WSIs). The pipeline includes WSI stain color normalization, ductal regions of interest (ROIs) segmentation, and cytological and architectural feature extraction to classify ductal ROIs into triaged high-risk benign lesions. The cytological feature extraction computes 196 morphological features such as roundness, aspect ratio, bounding box dimensions; intensity features such as means, variance, skewness, and kurtosis in multiple color channels and and texture features such as Haralick's features and graph run length features for each nuclei. In the architectural feature extraction, each ROI is represented by 5 different objects.

Dong Fei ET AL: "Computational Pathology to Discriminate Benign from Malignant Intraductal Proliferations of the Breast" (9 December 2014, ISSN: 1932-6203, DOI: 10.1371/journal.pone.0114885) describes a computational method for categorization of intraductal proliferative lesions of the breast based on routine light microscopic examination of histopathologic sections. The method includes nuclei segmentation and nuclei feature extraction. In the nuclei feature extraction first and second order statistical features are extracted. The first order statistical features determine the distribution of grey level values within nuclei regions and include mean, median, standard deviation, skewness and kurtosis. The second order statistical features include correlation, cluster shade, cluster prominence, energy, entropy, hara-correlation, homogeneity and inertia period.

Akif Burak Tosun ET AL: "Graph Run -Length Matrices for Histopathological Image Segmentation" in IEEE Transactions on Medical Imaging (March 2011) describes an algorithm for the segmentation of histopathological tissue images that includes the introduction of a new texture measure that models the spatial distribution of cytological tissue components and the use of this texture measure in histopathological image segmentation. In particular, the algorithm defines the texture of cytological components on a graph using the idea of grey-level run length matrices, and considers the runs of cytological components on the graph to form a run-length matrix, instead of considering the runs of pixel intensities.

### SUMMARY OF THE INVENTION

In one embodiment, a computational pathology method is provided that is broadly applicable to many histologies, including those relating to high-level and organ-specific disease entities, including both tumor and non-tumor pathology. The method includes receiving multi-parameter cellular and/or sub-cellular imaging data for an image of a tissue sample, and locating and segmenting a plurality of tissue components of the tissue sample in the multi-parameter cellular and sub-cellular imaging data to generate segmented multi-parameter cellular and sub-cellular imaging data. The method further includes applying a parametric feature modelling scheme to certain of the tissue components in the segmented multi-parameter cellular and sub-cellular imaging data, wherein the parametric feature modelling scheme is generated from a dictionary of pre-existing diagnostically relevant histological patterns and comprises a number of structural features adapted for defining a number of disease entities of a disease, and wherein the applying includes determining a quantification of each of the structural features for the tissue sample, and classifying a state of the disease in the tissue sample based the determined quantification of each of the structural features.

In another embodiment, a computerized computational pathology system for discriminating diagnostic tissue patterns in multi-parameter cellular and sub-cellular imaging data for a number of tissue samples from a number of patients or a number of multicellular in vitro models is provided. Like the method just described, the system is broadly applicable to many histologies, including those relating to high-level and organ-specific disease entities, including both tumor and non-tumor pathology. The system includes a processing apparatus, wherein the processing apparatus includes a number of components configured for: (i)locating and segmenting a plurality of tissue components of the tissue sample in the multi-parameter cellular and sub-cellular imaging data to generate segmented multi-parameter cellular and sub-cellular imaging data; (ii) applying a parametric feature modelling scheme to certain of the tissue components in the segmented multi-parameter cellular and sub-cellular imaging data, wherein the parametric feature modelling scheme is generated from a dictionary of pre-existing diagnostically relevant histological patterns and comprises a number of structural features adapted for defining a number of disease entities of a disease, and wherein the applying includes determining a quantification of each of the structural features for the tissue sample; and (iii) classifying a state of the disease in the tissue sample based the determined quantification of each of the structural features.

### BRIEF DESCRIPTION OF THE DRAWINGS

A full understanding of the invention can be gained from the following description of the preferred embodiments when read in conjunction with the accompanying drawings in which:
FIG. 1 is a schematic diagram of an exemplary digital pathology system for classifying tissue components in tissue samples according to an exemplary embodiment of the disclosed concept;
FIG. 2 is a flowchart illustrating a method of generating a parametric feature modeling scheme according to an exemplary embodiment of the disclosed concept;
FIG. 3 is a flowchart illustrating a method of classifying tissue components in tissue samples based on imaging data of the tissue samples and application of the parametric feature modeling scheme according to an exemplary embodiment of the disclosed concept;
FIG. 4 is a schematic diagram illustrating parametric models of histological patterns in the form of unary features according to an exemplary embodiment of the disclosed concept;
FIG. 5 is a schematic diagram illustrating parametric models of histological patterns in the form of binary features according to an exemplary embodiment of the disclosed concept;
FIG. 6 is a schematic diagram illustrating parametric models of histological patterns in the form of ternary features according to an exemplary embodiment of the disclosed concept;
FIG. 7 provides a table providing a listing of all the model parameters derived in connection with a particular exemplary embodiment of the disclosed concept;
FIG. 8 is a schematic diagram illustrating a methodology for computing likelihood scores to reveal dominant histological patterns according to an exemplary embodiment of the disclosed concept; and
FIG. 9 is a schematic diagram illustrating dominant histological patterns in representative images of low risk and high risk lesions according to an exemplary embodiment of the disclosed concept.

### DETAILED DESCRIPTION OF THE INVENTION

As used herein, the singular form of "a", "an", and "the" include plural references unless the context clearly dictates otherwise.

As used herein, the statement that two or more parts or components are "coupled" shall mean that the parts are joined or operate together either directly or indirectly, i.e., through one or more intermediate parts or components, so long as a link occurs.

As used herein, the term "number" shall mean one or an integer greater than one (i.e., a plurality).

As used herein, the terms "component" and "system" are intended to refer to a computer related entity, either hardware, a combination of hardware and software, software, or software in execution. For example, a component can be, but is not limited to being, a process running on a processor, a processor, an object, an executable, a thread of execution, a program, and/or a computer. By way of illustration, both an application running on a server and the server can be a component. One or more components can reside within a process and/or thread of execution, and a component can be localized on one computer and/or distributed between two or more computers. While certain ways of displaying information to users are shown and described with respect to certain figures or graphs as screenshots, those skilled in the relevant art will recognize that various other alternatives can be employed.

As used herein, the term "multi-parameter cellular and sub-cellular imaging data" shall mean data obtained from generating a number of images from a number of a sections of tissue which provides information about a plurality of measurable parameters at the cellular and/or sub-cellular level in the sections of tissue. Multi-parameter cellular and sub-cellular imaging data may be created by a number of different imaging modalities, such as, without limitation, any of the following: transmitted light (e.g., a combination of H&E and/or IHC (1 to multiple biomarkers)); fluorescence; immunofluorescence (including but not limited to antibodies and nanobodies and including but not limited to multiplexed (4-7) biomarkers and hyperplexed biomarkers (>7 biomarkers); live cell biomarkers multiplexing and/or hypeiplexing; electron microscopy, toponome imaging, matrix-assisted laser desorption/ionization mass spectrometric imaging (MALDI MSI), complementary spatial imaging (e.g., FISH, MxFISH, FISHSEQ, or CyTOF), multiparameter ion beam imaging, or in vitro imaging. Targets include, without limitation, tissue samples (human and animal) and in vitro models of tissues and organs (human and animal).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

The disclosed concept will now be described, for purposes of explanation, in connection with numerous specific details in order to provide a thorough understanding of the subject innovation. It will be evident, however, that the disclosed concept can be practiced without these specific details without departing from the scope of this innovation.

The disclosed concept provides a novel technological approach to computational pathology, using explainable AI (xAI). The xAI approach of the disclosed concept permits a completely different relationship between the pathologist and the software tool, one that permits transparency and accountability of the ML tool. The intent is to foster pathologist trust and acceptance of new and powerful computational tools. If an AI tool is to credibly support pathologist work on complex and difficult decisions, then it should be able to provide justifications and data for its conclusions. Having full situational awareness, the pathologist is empowered to make the very best diagnostic decisions that only they can make (e.g., benign versus malignant; high-risk versus low-risk; etc.).

Building on prior work in xAI tools of the present inventors, the disclosed concept provides a framework to parametrically model a dictionary of diagnostically relevant histological patterns and quantify their existence in digital pathology images. This framework will let pathologists visualize clinically relevant tissue structures in a quantitative fashion, given a parametric model. In the non-limiting exemplary embodiment, the disclosed concept accesses a potential dictionary of various histological patterns from several different public sources, including from the World Health Organization (WHO) classifications of tumors, and consultations with teams of different sub-specialist pathologist experts. These sources aid in the assembly of a comprehensive framework of high-level and organ-specific disease entities, including both tumor and non-tumor pathology, including breast, lung, gastrointestinal tract, skin, genitourinary tract, etc. These include: inflammatory infiltrate patterns (crypt abscesses in active colitis or presence of abnormal plasmacytoid dentritic cells in dermis), apoptosis in GI biopsies of transplant patients (GVHD is time-intensive), subtle non-tumor patterns in lung pathology, including looking for organism milieu before special stains are available, finding small tumor mets in almost any solid tumor, and finding incidental lymphomas in lymph nodes taken for solid tumor staging (CLL/SLL most commonly).

Many of these disease entities share feature patterns (e.g., gland formation in adenocarcinomas, duct formation in breast tissue), and others are disease-specific, such as colonic crypt distortion that is diagnostic of chronic mucosal ulcerative colitis. Using pre-existing classifications of disease, the approach of the disclosed concept may be generalized throughout multiple organ systems, thereby providing pathologists with relevant and disease-specific diagnostic recommendations that are explainable and justifiable using xAI techniques.

These guidelines would analytically model a visual pattern dictionary that traditionally defines the standards on tumor classification/nomenclature for pathologists worldwide. The models of the disclosed concept, built on WHO guidelines in the exemplary embodiment, will bring a solution to opaqueness when integrated with transparent and interpretable ML interfaces, hence promoting a better understanding of computational tools and tissue mechanisms.

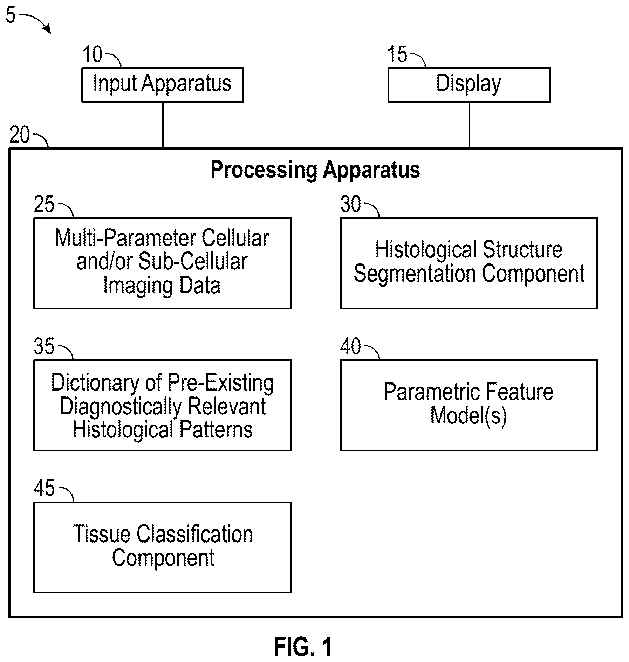

FIG. 1 is a schematic diagram of an exemplary digital pathology system 5 structured and configured for classifying tissue components in tissue samples (e.g., benign versus malignant; high-risk versus low-risk; etc.) based on a parametric feature modeling scheme according to an exemplary embodiment of the disclosed concept as described herein. As seen in FIG. 1, system 5 is a computing device structured and configured to generate and/or receive multi-parameter cellular and sub-cellular imaging data (labelled 25 in FIG. 1) and process that data as described herein to classify the state of a particular disease in the tissue sample through application of the parametric feature modeling scheme to the imaging data. System 5 may comprise, for example and without limitation, a PC, a laptop computer, a tablet computer, or any other suitable computing device structured and configured to perform the functionality described herein.

System 5 includes an input apparatus 10 (such as a keyboard), a display 15 (such as an LCD), and a processing apparatus 20. A user is able to provide input into processing apparatus 20 using input apparatus 10, and processing apparatus 20 provides output signals to display 15 to enable display 15 to display information to the user as described in detail herein (e.g., a segmented tissue image and a classification of a current disease state of certain tissue components in the tissue image). Processing apparatus 20 comprises a processor and a memory The processor may be, for example and without limitation, a microprocessor (µP), a microcontroller, an application specific integrated circuit (ASIC), or some other suitable processing device, that interfaces with the memory. The memory can be any one or more of a variety of types of internal and/or external storage media such as, without limitation, RAM, ROM, EPROM(s), EEPROM(s), FLASH, and the like that provide a storage register, i.e., a machine readable medium, for data storage such as in the fashion of an internal storage area of a computer, and can be volatile memory or nonvolatile memory. The memory has stored therein a number of routines that are executable by the processor, including routines for implementing the disclosed concept as described herein in various embodiments. In particular, processing apparatus 20 includes a histological structure segmentation component 30 configured for identifying and segmenting histological structures (such as, without limitation, ducts/glands and lumen, clusters of ducts/glands, and individual nuclei) in a number of tissue images represented by the multi-parameter cellular and/or sub-cellular imaging data 25 obtained from various imaging modalities as described herein in the various embodiments (e.g., H&E stained image data). In the non-limiting exemplary embodiment, histological structure segmentation component 30 employs the segmentation approach described in the United States Provisional Patent Application No. 62/990,264, titled "Scalable and High Precision Context Guided Segmentation of Histological Structures" and filed on March 16, 2020. That segmentation approach is able to locate and segment histological components, such as, without limitation, ducts, nuclei, blood vessels, lung alveoli, and colon glands.

Processing apparatus 20 further includes a dictionary of pre-existing diagnostically relevant visual histological patterns 35 that traditionally define the standards for classifying the state of a particular disease, such as breast cancer. In the non-limiting exemplary embodiment, the dictionary of pre-existing diagnostically relevant histological patterns is at least partially obtained from World Health Organization (WHO) Blue Books (including images contained therein). As is known in the art, WHO Blue Books are an essential standards reference for pathologists, clinicians and researchers internationally. The WHO Blue Books specify a body of knowledge regarding how histological patterns for differential diagnoses of diseases, such as tumors, can be described structurally with respect to (1) cell morphology (e.g., round, large, mitotic, etc.), (2) spatial cell organization (e.g., picket-fence, cribriform, etc.), and (3) architectural tissue organization (e.g., tumor infiltrating lymph nodes, fat at tumor boundary, etc.). All these histological patterns can be visually assessed by the pathologist, who typically arrives at a diagnosis by relating patterns in tissue samples to the patterns in the WHO standards.

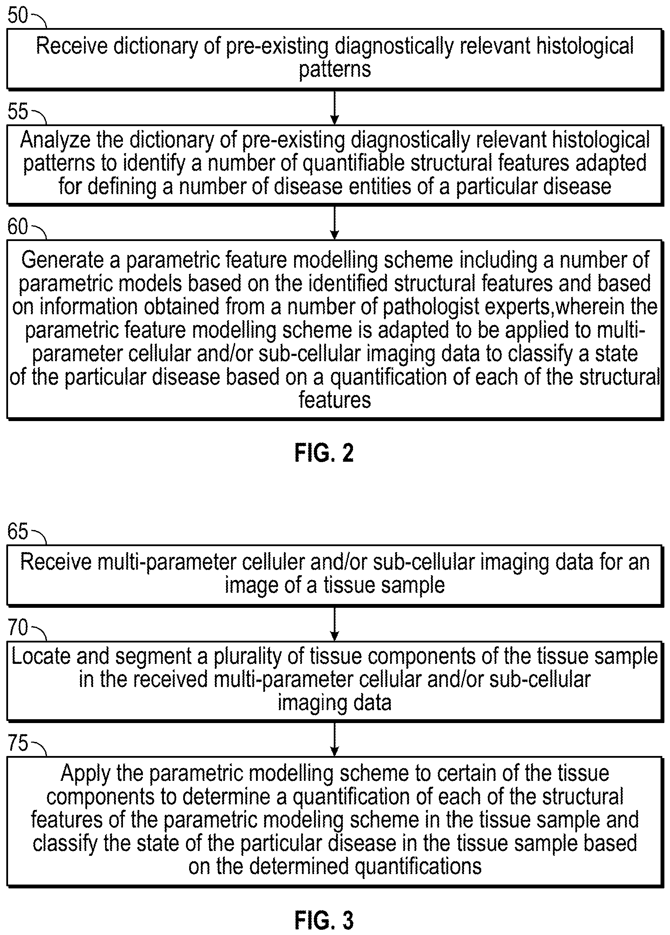

In addition, processing apparatus 20 further includes a number of parametric feature models that define a parametric feature modeling scheme that are derived from the dictionary of pre-existing diagnostically relevant histological patterns 35. One particular method for creating the number of parametric feature models 40 according to a particular exemplary embodiment is described in connection with FIG. 2 below. The parametric feature modeling scheme of the disclosed concept models the histological patterns from the dictionary of pre-existing diagnostically relevant histological patterns 35 and defines a number of quantifiable structural features that in turn may be used to define a number of disease entities for a number of particular diseases. As is known in the art, a disease entity is a set of features that define a classification of a disease, such as cancer. As such, the parametric feature modeling scheme analytically models the visual pattern dictionary that traditionally defines standards for classification of a disease.

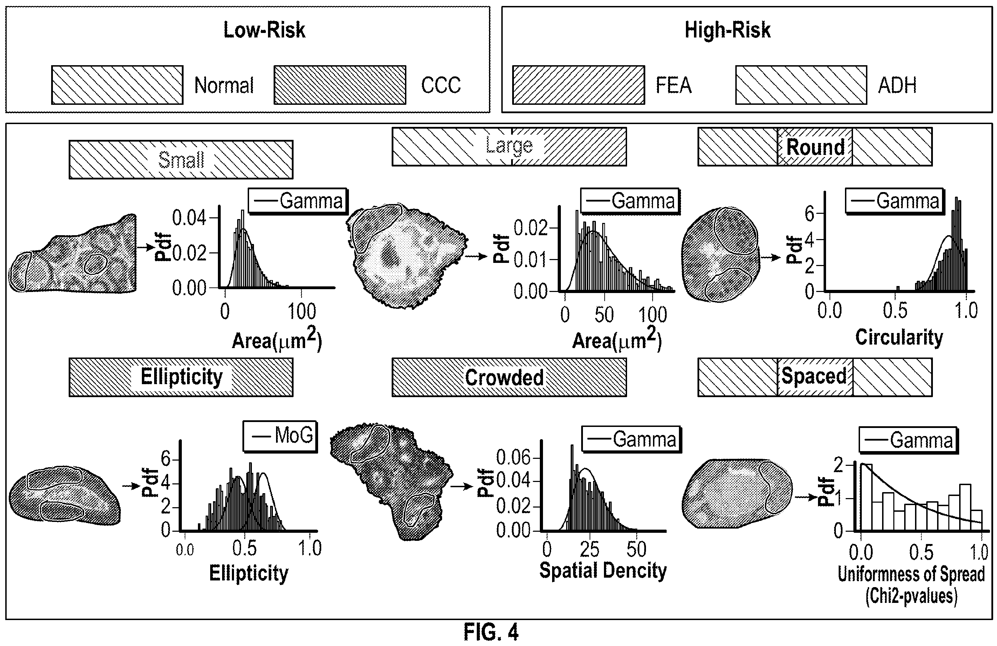

As described elsewhere herein, in the exemplary embodiment, the quantifiable features of the parametric feature modeling scheme may include one or more unary features, wherein each unary feature is a single morphological feature such as the size, shape or spatial spread of tissue components in an image, one or more binary features, wherein each binary feature is a pairwise combination of two unary features, and/or one or more ternary features, wherein each ternary feature is a combination of three or more unary features. Particular examples of such unary, binary and ternary features that may be employed in connection with one or more particular exemplary embodiments of the disclosed concept art described in detail elsewhere herein (FIGS. 4-6).

In addition, processing apparatus 20 also includes a tissue classification component 45. As described in detail elsewhere herein, tissue classification component 45 is structured and configured to apply one or more of the parametric feature models 40 to the multi-perimeter cellular and/or sub-cellular imaging data 25 in order to classify the state of a particular disease in a tissue sample represented by the multi-perimeter cellular and/or sub-cellular imaging data 25.

Referring to FIG. 2, a flowchart illustrating a method of generating the number of parametric feature models 40 according to an exemplary embodiment of the disclosed concept is provided. The method shown in FIG. 2 may be implemented in the form of one or more routines stored in and executable by the processing apparatus 20. The method begins at step 50, wherein the processing apparatus 20 receives the dictionary of pre-existing diagnostically relevant histological patterns 35. Next, at step 55, the method analyzes the dictionary of pre-existing diagnostically relevant histological patterns 35 to identify a number of quantifiable structural features that are adapted for defining a number of disease entities of a particular disease. In the exemplary embodiment, identification of the number of quantifiable structural features is based on information obtained by way of consultation with a number of pathologist experts. Then, at step 60, a parametric feature modeling scheme is generated that includes the number of parametric feature models 40. In step 60, in the non-limiting exemplary embodiment, the number of parametric feature models 40 is based on the identified structural features and information obtained by way of consultation with a number of pathologist experts. The parametric feature modeling scheme embodied by the number of parametric feature models 40 is adapted to be applied to the multi-perimeter cellular and/or sub- cellular imaging data 25 in order to classify a state of the particular disease in a tissue sample represented thereby based on a quantification of each of the structural features.

Referring to FIG. 3, a flowchart illustrating a method of classifying tissue components in tissue samples represented by the multi-perimeter cellular and/or subcellular imaging data 25 according to an exemplary embodiment of the disclosed concept is provided. The method shown in FIG. 3 may be implemented in the form of one or more routines stored in and executable by the processing apparatus 20. In the illustrated embodiment, the one or more routines form part of tissue classification component 45 shown in FIG. 1. The method of FIG. 3 begins at step 65, wherein the multi-perimeter cellular and/or sub-cellular imaging data 25 is received for at least image of a tissue sample in question. Next, at step 70, histological structure segmentation component 30 is employed to locate and segment a plurality of tissue components of the tissue sample in the received multi-perimeter cellular and/or subcellular imaging data 25. Then, at step 75, the parametric modeling scheme comprising the number of parametric feature models 40 is applied to certain of the tissue components represented by the multi-perimeter cellular and/or subcellular imaging data 25 to determine a quantification of each of the structural features of the parametric modeling scheme in that tissue sample. Step 75 also includes classifying the state of the particular disease in the tissue sample based on those determined quantifications.

Thus, as described in connection with FIGS. 1-3, the disclosed concept provides a system and method for parametric feature modeling of a pre-existing dictionary of histological patterns (obtained from digitized tissue samples) that define certain standards for disease classification, and for using the parametric feature modeling scheme to analyze digital pathology images to quantify the existence of certain features in the image and (thus the presence of one or more of the relevant histological patterns) and then classify the disease state of the image based on the feature values. As a result, the disclosed concept mimics the behavior that clinical pathologists employ when arriving at diagnoses using visual histological patterns that are accepted as standards for defining disease classification.

The disclosed concept will now, for illustrative purposes, be described in connection with one particular exemplary embodiment that is an approach for analyzing and classifying breast lesions. More specifically, this particular exemplary embodiment employs a particular parametric feature modeling scheme that is described in detail below in order to allow for the automatic classification of tumors in the relevant breast lesion images. In this particular exemplary embodiment, step 70 of FIG. 3 (i.e., the tissue component location and segmentation step) is implemented using the duct and nuclei segmentation approach described in the previously identified provisional patent application. It will be understood, however, that this is meant to be exemplary only and that the disclosed concept may be used for analyzing and classifying the disease state of other diseases in tissue sample images.

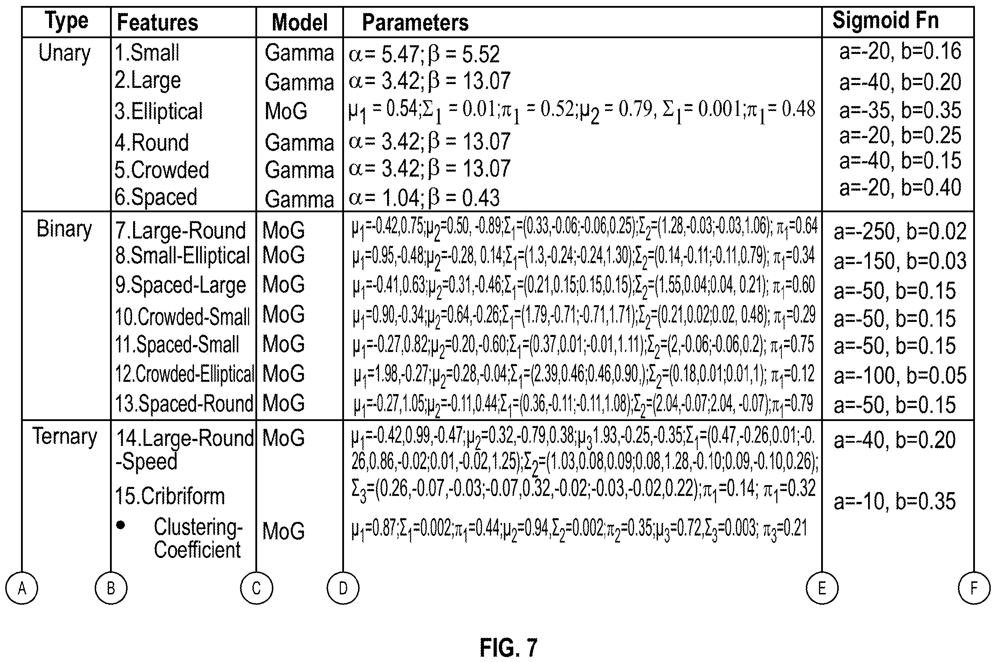

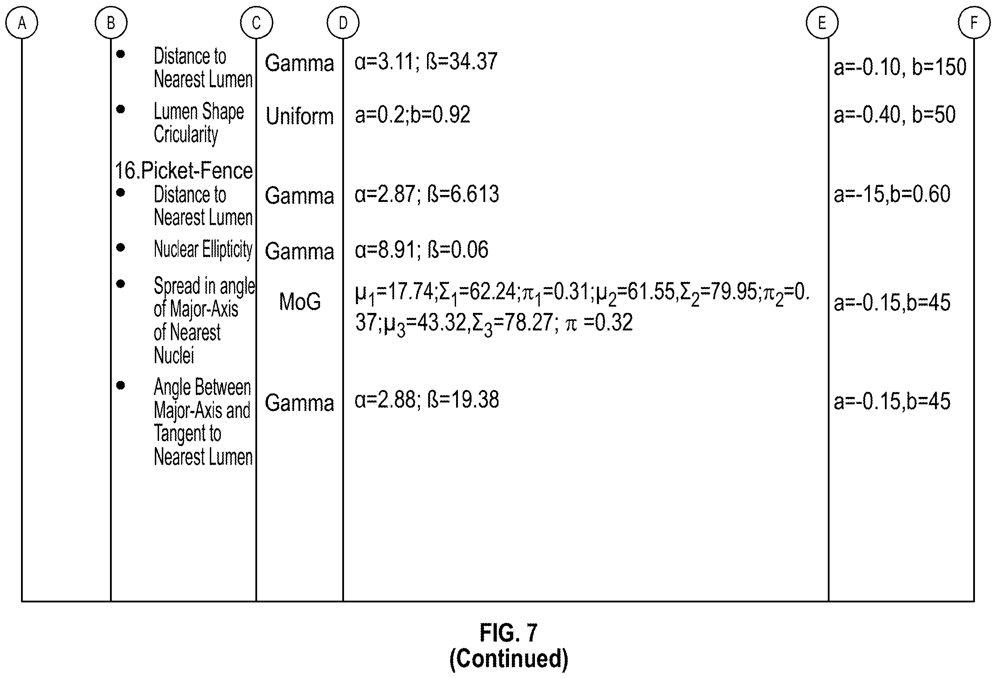

The particular embodiment for analyzing and classifying breast lesions described herein invokes a parametric feature model(s) 40 for histological patterns within each segmented duct using a mix of unary, binary, and ternary features as shown in FIGS. 4-6. More specifically, in this particular embodiment, the disclosed concept defines three types of decompositions, since cells and tissue components may carry multiple features together. The hatched bars over each feature in FIGS. 4-6 indicate the lesion where the feature is most likely to be found, e.g., large and round nuclei are often found in high-risk lesions, small and elliptical nuclei are often found in low-risk lesions, and cribriform patterns tend to be exclusive to atypical ductal hyperplasia (ADH). FIG. 7 provides a table providing a listing of all the model parameters derived in connection with this particular embodiment.

The unary features of this particular embodiment are shown in FIG. 4. As seen in FIG. 4 and described below, such unary features include a spectrum of morphological features on the basis of size, shape, and spatial spread around each nucleus.

The first group of unary features of this embodiment (the "smallness" and "largeness" features) are based on nuclear size (quantified using area), which is known to provide diagnostic cues in pathological grading, with groups of small and large nuclei having a propensity to belong to low-risk and high-risk lesions, respectively, as shown in FIG. 4. To build analytical models of small and large features, the disclosed concept first constructs a histogram of nuclear areas obtained from an ensemble of regions of interest (ROIs) showing prototypical example regions within a duct containing small and large nuclei (FIG. 4), and then models this histogram with a Gamma distribution.

The second group of unary features of this embodiment (the "roundness" and "ellipticity" features) are based on nuclear shape, which has been identified as diagnostically meaningful. For example, as shown in FIG. 4, columnar cell change (CCC) lesions are known to show dominant elliptical nuclei. Thus, in an aspect of this particular embodiment, the disclosed concept quantitates these features with a roundness feature measured as (4π×area)/perimeter² and an ellipticity feature given by the ratio of the length of the minor-axis to the length of major-axis. Roundness ranges from 0 (irregular star-like appearance) to 1 (perfect circle), while ellipticity characterizes the "flatness" of an object, with lower values denoting highly elliptical nuclei (FIG. 4). In each case, because of the intrinsic heterogeneity of these measurements, the disclosed concept considers a spatial neighborhood around each nucleus, and models the distributions of roundness with a Gamma distribution and ellipticity with a 2-component mixture of Gaussians (MoG) model (FIG. 4).

Moreover, several studies have shown that studying the spatial organization of nuclei provides insights into the abnormalities of cells which might eventually lead to malignancy. For instance, the nuclei arrangement in a CCC lesion frequently exhibits crowding and/or overlapping. However, for cases belonging to high-risk atypical lesions (Flat Epithelial Atypia-FEA and ADH), the nuclei tend to be uniform and evenly-spaced. Thus, the third group of unary features of this embodiment (the "crowdedness" and "spacedness" features) are based on the spatial organization of nuclei in an image. To quantify the crowding around each nucleus, its average distance to ten nearest nuclei is computed. An analytical model of crowdedness is then constructed by considering local ROIs within a duct where clusters of nuclei show significant crowding behavior and then computing its spatial density. In contrast, to capture evenly spaced/uniform dispersion patterns around a nucleus, the disclosed concept starts by placing a regular grid of size 3×3 centered at a reference nucleus, and measures the density of twenty neighboring nuclei by counting the population of nuclei in each grid cell as described in Sergio Rey, Wei Kang, Hu Shao, Levi John Wolf, Mridul Seth, James Gaboardi, and Dani Arribas-Bel, "pointpats: Point Pattern Analysis in PySAL", PySAL: The Python Spatial Analysis Library, July 2019. The disclosed concept then compares this observed population against an expected number of nuclei under the *complete spatial randomness* hypothesis, which asserts the occurrence of points (here nuclei) within grids in a random fashion analogous to a Poisson point process using a an χ²-test statistic and acquiring the corresponding p-value using the χ² distribution table. The larger the p-value, the greater is the likelihood of observing a uniform/evenly spaced dispersion of nuclei around the reference nucleus.

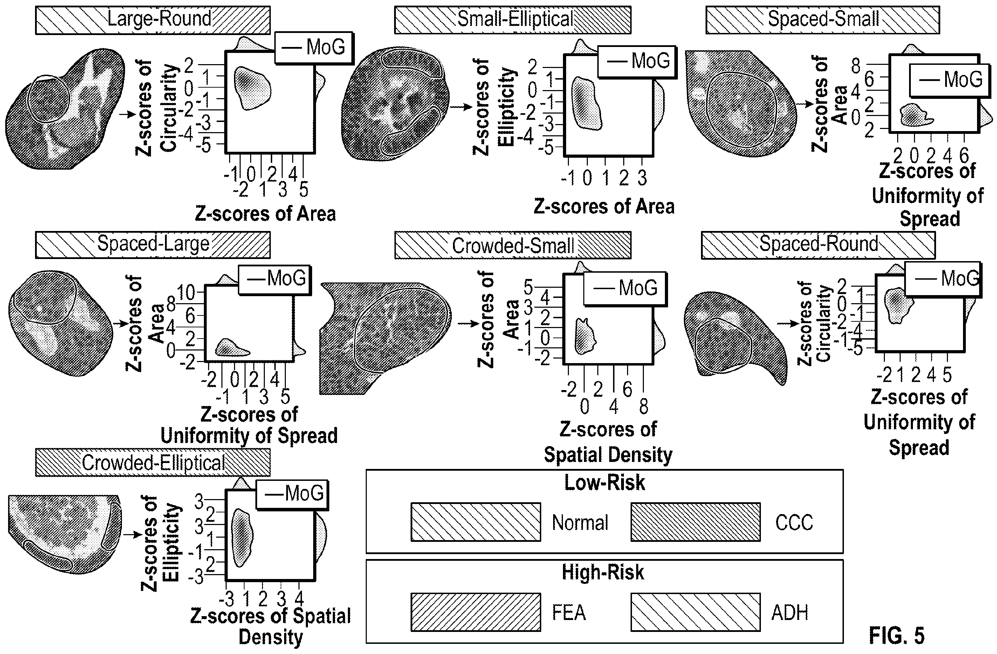

Although, the unary features described above show some inferential strength (indicated by the hatched bars on top of each feature in FIG. 4), a pathologist typically makes an informed decision by paying attention to the pairwise combinations of such features. For instance, a CCC lesion (low-risk) exhibits a crowded and elliptical nuclei arrangement, whereas a high-risk lesion tends to display a greater likelihood of large-round, spaced-large, and spaced-round nuclei. Furthermore, a lesion showing majority regions of small nuclei coupled with crowded and/or spaced behavior is representative of a normal duct. Thus, this embodiment of the disclosed concept considers seven such binary features obtained from pairwise combinations of unary features, which is shown in FIG. 5. In particular, as seen in FIG. 5, the binary features of the present exemplary embodiment include a "nuclear largeness-roundness" feature, a "nuclear smallness-ellipticity" feature, a "nuclear spacedness-largeness" feature, a "nuclear crowdedness-smallness" feature, a "nuclear spacedness-smallness" feature, a "nuclear crowdedness-ellipticity" feature, and a "nuclear spacedness-roundness feature." In the exemplary implementation, to generate the binary features, the disclosed concept takes the z-scores for each unary feature and models the joint distribution of z-scores from the feature pair with a two-component, two-dimensional mixture of Gaussian distribution.

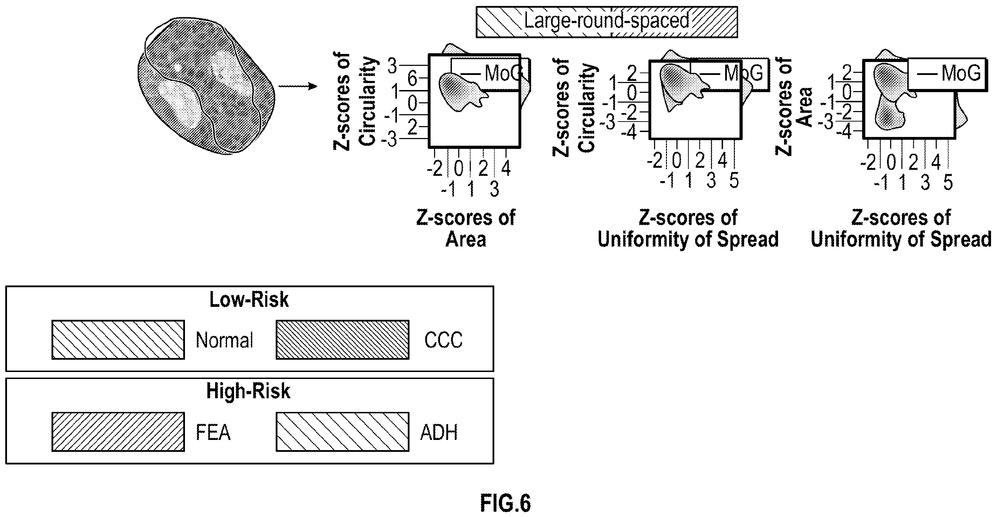

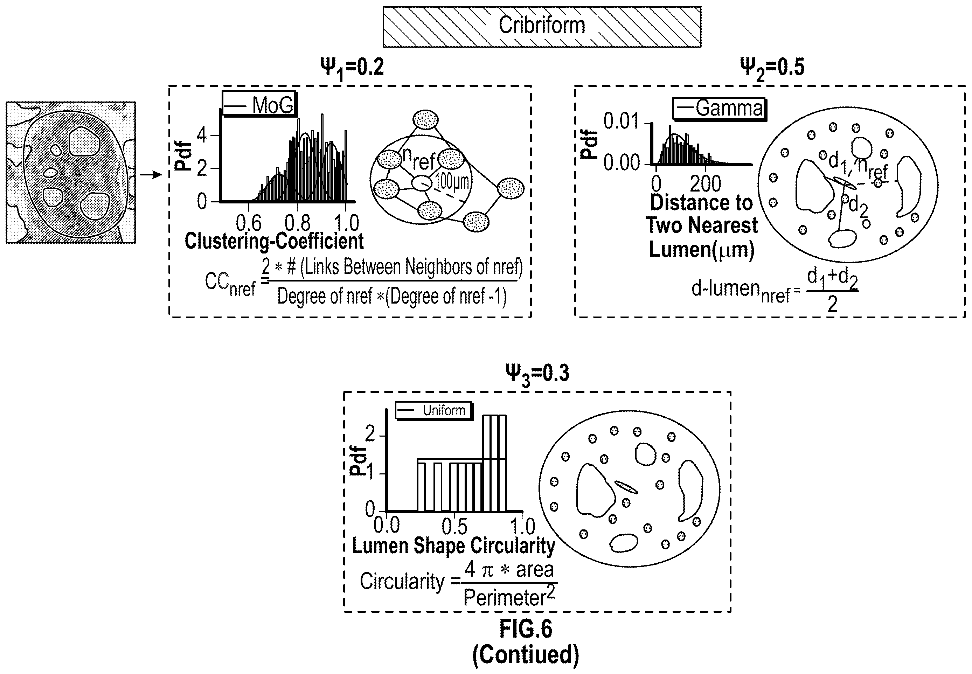

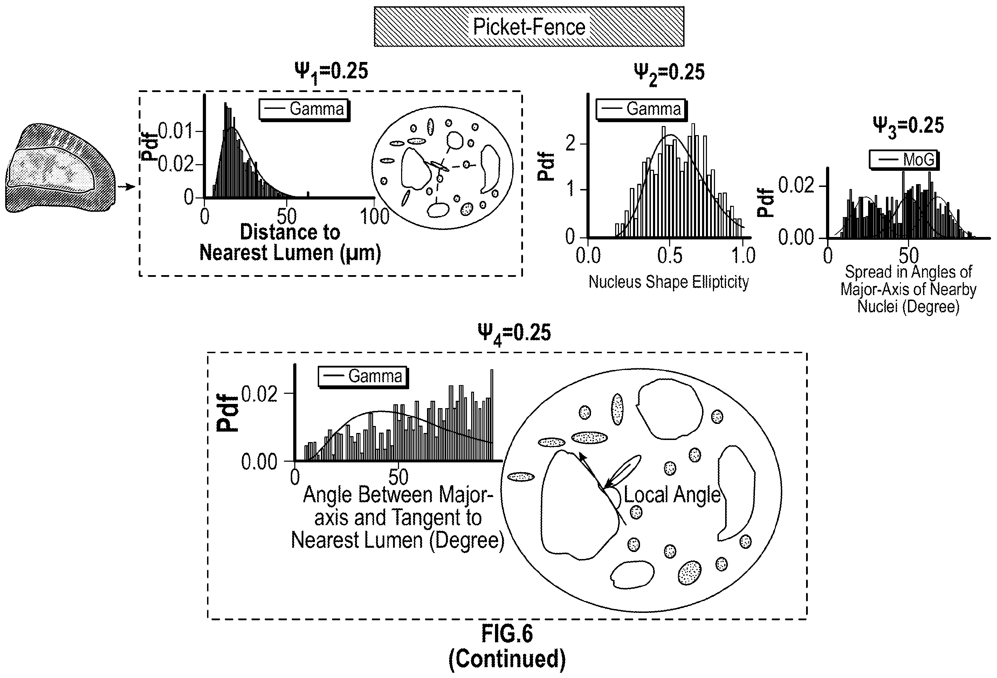

Moreover, some of the diagnostically relevant histological patterns are best represented by a combination of more than two unary features. This embodiment of the disclosed concept, therefore, considers three such ternary features obtained from combinations of more than two unary features (including, but limited to the specific unary features described above), which is shown in FIG. 6. As seen in FIG. 6, the ternary features of the present exemplary embodiment include a "nuclear largeness-roundness-spacedness" feature, a "cribriform" feature and a "picket-fence feature."

In particular, to determine the largeness-roundness-spacedness feature, the disclosed concept takes z-scores from each unary feature, i.e., largeness, roundness and spacedness, and builds a three-component, three-dimensional mixture of Gaussian model using ground truth examples.

With respect to the cribriform feature, this pattern is characterized by polarization of epithelial cells within spaces formed by "almost" circular multiple lumen (> 2) which are 5-6 cells wide and whose appearance closely resemble "holes in Swiss cheese". This complex architectural pattern can be identified by analytically modeling three (unary) sub-features: clustering coefficient, distance of the nucleus from two nearest lumen, and circularity of the lumen adjacent to the nucleus. The polarization of epithelial cells around the lumen is characterized by a clustering coefficient and is computed by following the method described in Naiyun Zhou, Andrey Fedorov, Fiona Fennessy, Ron Kikinis, and Yi Gao, Large Scale Digital Prostate Pathology Image Analysis Combining Feature Extraction and Deep Neural Network, arXiv preprint arXiv: 1705.02678, 2017, and is illustrated in the middle row of FIG. 6. Furthermore, a group of nuclei occupying the spacing between two lumen has a tendency to show a cribriform pattern around them. Thus, the disclosed concept measures the average distance between each nucleus to the nearest two lumen and models its distribution using a gamma function (see middle row of FIG. 6). The final likelihood for a cribriform pattern is obtained from the weighted sum of the likelihood scores of sub-features. In this aspect, the disclosed concept performed a grid search on the mixing coefficients to learn that the likelihood scores from the three sub-features should, in the exemplary embodiment, be mixed in the proportion of 0.2, 0.5, and 0.3 respectively.

With respect to the picket-fence feature, this pattern is recognized from a group of crowded elliptical nuclei oriented perpendicular to the basement membrane (lumen). The analytical model of this high-order visual feature can be obtained by constructing parametric models of four simple (unary) sub-features: distance of a nucleus to nearest lumen, nuclear ellipticity, a spread in the angle of major-axis of 10 nearby nuclei, and its local angle with respect to the basement membrane as shown in the last row of FIG. 6. Since, each sub-feature contributes equally to observing this ternary feature, in the exemplary embodiment, the disclosed concept assigns a mixing coefficient of 0.25 in combining the likelihood scores from the four sub-features to determine the presence of a picket-fence pattern.

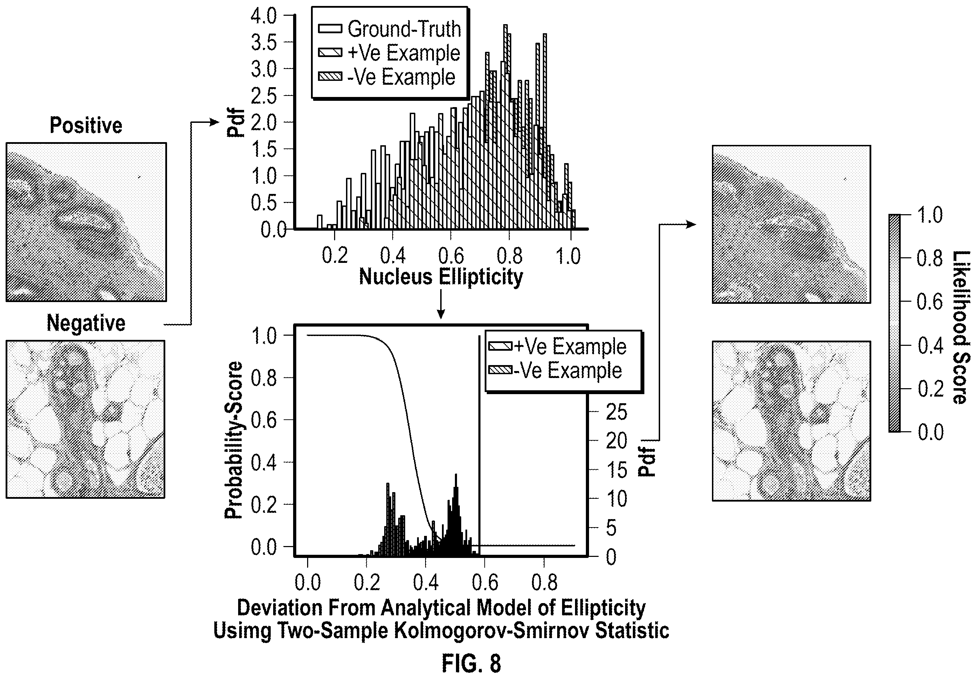

As discussed above, the parametric models for the histological patterns are, in the exemplary embodiment, probability distributions. For example, a cytological feature like nuclear ellipticity for a given nucleus inside a ROI will receive a probability under the mixture of Gaussian models shown in Fig. 4. However, this feature can be made diagnostically more powerful by considering its spatial context, i.e., an elliptical nucleus is typically surrounded by other elliptically shaped nuclei. In one aspect, the disclosed concept assesses this by constructing first a distribution of the feature values from all nuclei found within a radius of 100µm of the reference nucleus (hyperparameter optimized by trial and error) and comparing this to the canonical distribution derived from ground-truth examples used to derive the parametric models. The disclosed concept use two distance measures where appropriate: Kullback-Leibler divergence for mixture of Gaussians and the two-sample Kolmogorov Smirnov test for unimodal Gamma distributions derived as described previously. Small distances imply greater evidence for the pattern. The disclosed concept turns the distances into a likelihood score by an inverted S-function as shown in FIG. 8.

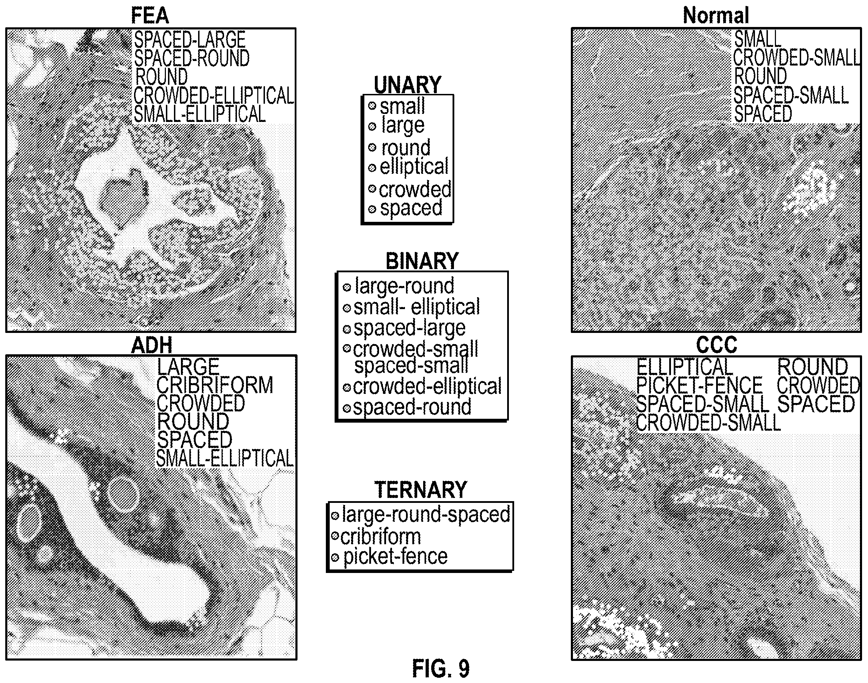

With respect to a strategy for differential diagnosis, the disclosed concept, in the exemplary embodiment, adopts a non-linear strategy, similar to what expert pathologists do, in that it finds sub-regions within ROI by non-maxima suppression (threshold value of 0.85 on the likelihood scores) where the evidence for one or more of the unary, binary, or ternary feature is dominating. FIG. 9 provides a visual illustration of the likelihood maps of dominant patterns in representative images of low- and high-risk lesions. Low-risk lesions show dominant islands of round, small, spaced, and spaced-small in a normal ROI and elliptical, round, spaced-small, crowded-small, and picket-fence neighborhoods in a CCC ROI. In comparison, high-risk lesions show dominant regions of spaced-large, and spaced-round in an FEA labeled ROI- and compelling strengths for large and cribriform patterns along with traces of crowded and spaced in ADH labeled ROI. These patterns validate the canonical forms shown in FIGS. 4-6.

Furthermore, having identified dominant unary, binary and ternary feature regions, the disclosed concept, in the exemplary embodiment, uses three descriptive statistics: median value of the likelihood scores of all the nuclei found in each sub-region, median number of nuclei found in each sub-region and the number of sub-regions. This is calculated for each one of the unary, binary and ternary features (total = 16), thereby obtaining a 48 column feature vector for a single image. In the exemplary embodiment, feature vectors were computed for all 1441 labeled duct ROIs extracted from whole slide images which resulted in 834×48 size feature map used to train a classifier and 607×48 data matrix for testing. To analyze the benefit of including binary and ternary features, the disclosed concept further slices the 48 column feature vector to be suitable for three scenarios: unary (U) only, unary and binary (U-B), and unary, binary, and ternary features (U-B-T). Due to inherent training and testing class imbalance, which reflects the real-world prevalence statistics of atypical lesions, the disclosed concept up-sampled high-risk examples using the SMOTE technique as described in Chawla N. et al., "Smote: Synthetic Minority Over-sampling Technique", Journal of Artificial Intelligence Research, 16:321-357, 2002.

In addition, prior to classifying the lesions, the disclosed concept pays close attention to the presence of a cribriform pattern, a symbolic visual primitive of an ADH (a high-risk) category. ROIs predicted to show a cribriform pattern are classified as high-risk, if the number of nuclei forming the cribriform sub-region is greater than 8 (hyper-parameter optimized over the training data). The reduced dataset, devoid of cribriform, is tested for each of the scenarios (U, U-B, and U-B-T) with logistic regression (LR), support vector machine (SVM), random forest (RF), and gradient boosted classifier algorithms. The best model was chosen by optimizing the parameters using GridSearchCV based on precision, recall, and F-scores and then performed a 10-fold stratified cross-validation to check for overfitting.

The approach of this particular embodiment of the disclosed concept as just described, with ~150 parameters (see FIG. 7) is readily amenable to being explained and cannot be delivered by current deep learning (DL) methods, which typically require ~10-50 million parameters and large training data. Moreover, there appears to be no widely reported DL methods for analysis or classification of tough pathologies, such as of atypical breast lesions, adenomatous polyps in colon, or idiopathic pulmonary fibrosis in lung, but an abundance of these algorithms for cancer vs no-cancer datasets.

While specific embodiments of the invention have been described in detail, it will be appreciated by those skilled in the art that various modifications and alternatives to those details could be developed in light of the overall teachings of the disclosure. Accordingly, the particular arrangements disclosed are meant to be illustrative only and not limiting as to the scope of disclosed concept which is to be given the full breadth of the claims appended and any and all equivalents thereof.

## Claims

1. A computer-implemented pathology method, comprising:
receiving multi-parameter cellular and/or sub-cellular imaging data for an image of a tissue sample;
locating and segmenting a plurality of tissue components of the tissue sample in the multi-parameter cellular and sub-cellular imaging data to generate segmented multi-parameter cellular and sub-cellular imaging data; and
applying a parametric feature modelling scheme to certain of the tissue components in the segmented multi-parameter cellular and sub-cellular imaging data, wherein the parametric feature modelling scheme is generated from a dictionary of pre-existing diagnostically relevant histological patterns and comprises a number of structural features adapted for defining a number of disease entities of a disease, wherein the number of structural features comprises a number of unary features wherein each of the unary features is a single morphological feature, a number of binary features wherein each of the binary features is a pairwise combination of two of the unary features, and a number of ternary features wherein each ternary feature is a combination of three or more features selected from the unary features or other structural features, and wherein the applying includes determining a quantification of each of the structural features for the tissue sample; and
classifying a state of the disease in the tissue sample based the determined quantification of each of the structural features.

2. A computerized computational pathology system for discriminating diagnostic tissue patterns in multi-parameter cellular and sub-cellular imaging data for a number of tissue samples from a number of patients or a number of multicellular in vitro models, comprising:
a processing apparatus, wherein the processing apparatus includes a number of components configured for:
locating and segmenting a plurality of tissue components of the tissue sample in the multi-parameter cellular and sub-cellular imaging data to generate segmented multi-parameter cellular and sub-cellular imaging data;
applying a parametric feature modelling scheme to certain of the tissue components in the segmented multi-parameter cellular and sub-cellular imaging data, wherein the parametric feature modelling scheme is generated from a dictionary of pre-existing diagnostically relevant histological patterns and comprises a number of structural features adapted for defining a number of disease entities of a disease, wherein the number of structural features comprises a number of unary features wherein each of the unary features is a single morphological feature, a number of binary features wherein each of the binary features is a pairwise combination of two of the unary features, and a number of ternary features wherein each ternary feature is a combination of three or more features selected from the unary features or other structural features, and wherein the applying includes determining a quantification of each of the structural features for the tissue sample; and
classifying a state of the disease in the tissue sample based the determined quantification of each of the structural features.

3. The method according to claim 1 or the system according to claim 2, wherein the structural features include a number of cell morphology features and a number of spatial cell organization features.

4. The method or system according to claim 3, wherein the locating and segmenting the plurality of tissue components comprises locating and segmenting a plurality of nuclei in the tissue sample, the certain of the tissue components comprising the plurality of nuclei, and wherein the number of cell morphology features includes a number of size features each based on a nuclear size of each of the nuclei, a number of shape features each based on a nuclear shape of each of nuclei, and a number of spatial spread features each based on a degree of nuclear spacing of each of the nuclei.

5. The method or system according to claim 4, wherein the number of size features includes a nuclear smallness feature and a nuclear largeness feature, wherein the number of shape features includes a nuclear roundness feature and a nuclear ellipticity feature, and wherein the number of spatial spread features includes a nuclear crowdedness feature and a nuclear spacedness feature.

6. The method or system according to claim 5, wherein:
(i) the nuclear smallness feature is based on a first histogram of nuclear areas obtained from prototypical regions containing nuclei of a first size classification comprising a small classification, wherein the first histogram is modelled with a Gamma distribution, and wherein the nuclear largeness feature is based on a second histogram of nuclear areas obtained from prototypical regions containing nuclei of a second size classification comprising a large classification, wherein the second histogram is modelled with a Gamma distribution; or
(ii) the nuclear crowdedness feature is quantified by computing, for each nucleus, an average distance to a plurality of nearest neighbor nuclei; or
(iii) the nuclear spacedeness feature is quantified by, for each nucleus, placing a grid cell centered at a reference nucleus and measuring a density of a plurality of neighboring nuclei by counting a population of nuclei in the grid cell.

7. The method or system according to claim 5, wherein the nuclear roundness feature is based on a number of first measurements each given by (4π×area)/perimeter² and wherein the nuclear ellipticity feature is based on a number of second measurements each given a ratio of a length of a minor-axis to a length of a major-axis.

8. The method or system according to claim 7, wherein the nuclear roundness feature ranges from 0 which is indicative of an irregular star-like appearance to 1 which is indicative of a perfect circle, and wherein the nuclear ellipticity feature characterizes a flatness of a nucleus wherein lower values denote highly elliptical nuclei.

9. The method according to claim 7, wherein the nuclear roundness feature considers a spatial neighborhood around each nucleus and models the distributions of roundness with a Gamma distribution, and wherein the nuclear ellipticity feature considers a spatial neighborhood around each nucleus and models the distributions of ellipticity with a 2-component mixture of Gaussians (MoG) model.

10. The method or system according to claim 6, wherein the population is compared against an expected number of nuclei under a complete spatial randomness hypothesis.

11. The method or system according to claim 5, wherein the number of ternary features includes a cribriform feature indicative of a degree to which the certain of the tissue components exhibit a cribriform pattern and a picket-fence feature indicative of a degree to which the certain of the tissue components exhibit a picket-fence pattern.

12. The method according to claim 1 or the system according to claim 2, wherein each binary feature comprises a joint distribution of z-scores from the unary features thereof with a two-component, two-dimensional mixture of Gaussian distribution.

13. The method or system according to claim 11, wherein the number of unary features comprises the nuclear smallness feature, the nuclear largeness feature, the nuclear roundness feature, the nuclear ellipticity feature, the nuclear crowdedness feature, and the nuclear spacedness feature, wherein the number of binary features includes a nuclear largeness-roundness feature, a nuclear smallness-ellipticity feature, a nuclear spacedness-largeness feature, a nuclear crowdedness-smallness feature, a nuclear spacedness-smallness feature, a nuclear crowdedness-ellipticity feature, and a nuclear spacedness-roundness feature, and wherein the number of ternary features includes a nuclear largeness-roundness-spacedness feature, the cribriform feature and the picket-fence feature.

14. The method according to claim 1 or the system according to claim 2, wherein the number of disease entities comprise a number of organ-specific disease entities, including both tumor and non-tumor pathology.

15. A non-transitory computer readable medium storing one or more programs, including instructions, which when executed by a computer, causes the computer to perform the method of claim 1.

## Patentansprüche

1. Computerimplementiertes Pathologieverfahren, umfassend:
Empfangen von zellulären und/oder subzellulären Mehrparameter-Bildgebungsdaten für ein Bild einer Gewebeprobe;
Lokalisieren und Segmentieren einer Vielzahl von Gewebekomponenten der Gewebeprobe in den zellulären und subzellulären Mehrparameter-Bildgebungsdaten, um segmentierte zelluläre und subzelluläre Mehrparameter-Bildgebungsdaten zu erzeugen; und
Anwenden eines parametrischen Merkmalmodellierungsschemas auf bestimmte der Gewebekomponenten in den segmentierten zellulären und subzellulären Mehrparameter-Bildgebungsdaten, wobei das parametrische Merkmalmodellierungsschema aus einer Sammlung bereits bestehender diagnostisch relevanter histologischer Muster erzeugt wird und eine Anzahl von Strukturmerkmalen umfasst, die ausgelegt sind, um eine Anzahl von Erkrankungsgebilde einer Erkrankung zu definieren, wobei die Anzahl von Strukturmerkmalen eine Anzahl von unären Merkmalen, wobei jedes der unären Merkmale ein einzelnes morphologisches Merkmal ist, eine Anzahl von binären Merkmalen, wobei jedes der binären Merkmale eine paarweise Kombination von zwei der unären Merkmalen ist, und eine Anzahl von ternären Merkmalen umfasst, wobei jedes ternäre Merkmal eine Kombination aus drei oder mehr Merkmalen ist, die aus den unären Merkmalen oder anderen Strukturmerkmalen ausgewählt sind, und wobei das Anwenden das Bestimmen einer Quantifizierung jedes der Strukturmerkmale für die Gewebeprobe umfasst; und
Klassifizieren eines Zustands der Erkrankung in der Gewebeprobe basierend auf der bestimmten Quantifizierung jedes der Strukturmerkmale.

2. Computerisiertes Computerpathologiesystem zum Unterscheiden diagnostischer Gewebemuster in zellulären und subzellulären Mehrparameter-Bildgebungsdaten für eine Anzahl von Gewebeproben von einer Anzahl von Patienten oder einer Anzahl von multizellulären In-vitro-Modellen, umfassend:
eine Verarbeitungsvorrichtung, wobei die Verarbeitungsvorrichtung eine Anzahl von Komponenten umfasst, die ausgelegt sind zum:
Lokalisieren und Segmentieren einer Vielzahl von Gewebekomponenten der Gewebeprobe in den zellulären und subzellulären Mehrparameter-Bildgebungsdaten, um segmentierte zelluläre und subzelluläre Mehrparameter-Bildgebungsdaten zu erzeugen;
Anwenden eines parametrischen Merkmalmodellierungsschemas auf bestimmte der Gewebekomponenten in den segmentierten zellulären und subzellulären Mehrparameter-Bildgebungsdaten, wobei das parametrische Merkmalmodellierungsschema aus einer Sammlung bereits bestehender diagnostisch relevanter histologischer Muster erzeugt wird und eine Anzahl von Strukturmerkmalen umfasst, die ausgelegt sind, um eine Anzahl von Erkrankungsgebilde einer Erkrankung zu definieren, wobei die Anzahl von Strukturmerkmalen eine Anzahl von unären Merkmalen, wobei jedes der unären Merkmale ein einzelnes morphologisches Merkmal ist, eine Anzahl von binären Merkmalen, wobei jedes der binären Merkmale eine paarweise Kombination von zwei der unären Merkmalen ist, und eine Anzahl von ternären Merkmalen umfasst, wobei jedes ternäre Merkmal eine Kombination aus drei oder mehr Merkmalen ist, die aus den unären Merkmalen oder anderen Strukturmerkmalen ausgewählt sind, und wobei das Anwenden das Bestimmen einer Quantifizierung jedes der Strukturmerkmale für die Gewebeprobe umfasst; und
Klassifizieren eines Zustands der Erkrankung in der Gewebeprobe basierend auf der bestimmten Quantifizierung jedes der Strukturmerkmale.

3. Verfahren nach Anspruch 1 oder System nach Anspruch 2, wobei die Strukturmerkmale eine Anzahl von Zellmorphologiemerkmalen und eine Anzahl von räumlichen Zellorganisationsmerkmalen umfassen.

4. Verfahren oder System nach Anspruch 3, wobei das Lokalisieren und Segmentieren der Vielzahl von Gewebeproben das Lokalisieren und Segmentieren einer Vielzahl von Zellkernen in der Gewebeprobe umfasst, wobei die bestimmten der Gewebekomponenten die Vielzahl von Zellkernen umfassen, und wobei die Anzahl von Zellmorphologiemerkmalen eine Anzahl von Größenmerkmalen, von denen jedes auf einer Zellkerngröße jedes der Zellkerne basiert, eine Anzahl von Formmerkmalen, von denen jedes auf einer Zellkernform jedes der Zellkerne basiert, und eine Anzahl von Raumverteilungsmerkmalen, von denen jedes auf einem Grad der Zellkernbeabstandung jedes der Zellkerne basiert, umfasst.

5. Verfahren oder System nach Anspruch 4, wobei die Anzahl von Größenmerkmalen ein Zellkernkleinheitsmerkmal und ein Zellkerngrößenmerkmal umfasst, wobei die Anzahl von Formmerkmalen ein Zellkernrundheitsmerkmal und ein Zellkernelliptizitätsmerkmal umfasst und wobei die Anzahl von Raumverteilungsmerkmalen ein Zellkernballungsmerkmal und ein Zellkernbeabstandungsmerkmal umfasst.

6. Verfahren oder System nach Anspruch 5, wobei:
(i) das Zellkernkleinheitsmerkmal auf einem ersten Histogramm von Zellkernbereichen basiert, die aus prototypischen Regionen erhalten wurden, die Zellkerne einer ersten Größenklassifikation, umfassend eine kleine Klassifikation, enthalten, wobei das erste Histogramm mit einer Gamma-Verteilung modelliert ist, und wobei das Zellkerngrößenmerkmal auf einem zweiten Histogramm von Zellkernbereichen basiert, die aus prototypischen Regionen erhalten wurden, die Zellkerne einer zweiten Größenklassifikation, umfassend eine große Klassifikation, enthalten, wobei das zweite Histogramm mit einer Gamma-Verteilung modelliert ist; oder
(ii) das Zellkernballungsmerkmal durch Berechnen für jeden Zellkern eines mittleren Abstands zu einer Vielzahl von nächsten benachbarten Zellkernen quantifiziert wird; oder
(iii) das Zellkernbeabstandungsmerkmal für jeden Zellkern durch Platzieren einer Rasterzelle, die an einem Referenzzellkern zentriert ist, und Messen einer Dichte einer Vielzahl von benachbarten Zellkernen durch Zählen einer Population von Zellkernen in der Rasterzelle quantifiziert wird.

7. Verfahren oder System nach Anspruch 5, wobei das Zellkernrundheitsmerkmal auf einer Anzahl von ersten Messungen basiert, die jeweils durch (4π x Fläche)/Perimeter² angegeben ist, und wobei das Zellkernelliptizitätsmerkmal auf einer Anzahl von zweiten Messungen basiert, die jeweils durch ein Verhältnis einer Länge einer kleinen Achse zur Länge einer großen Achse angegeben ist.

8. Verfahren oder System nach Anspruch 7, wobei das Zellkernrundheitsmerkmal im Bereich von 0, was ein unregelmäßiges sternähnliches Erscheinungsbild angibt, bis 1, was einen perfekten Kreis angibt, liegt, und wobei das Zellkernelliptizitätsmerkmal eine Flachheit eines Zellkerns kennzeichnet, wobei niedrigere Werte stark elliptische Zellkerne bezeichnen.

9. Verfahren nach Anspruch 7, wobei das Zellkernrundheitsmerkmal eine räumliche Nachbarschaft rund um jeden Zellkern berücksichtigt und die Verteilungen der Rundheit mit einer Gamma-Verteilung modelliert, und wobei das Zellkernelliptizitätsmerkmal eine räumliche Nachbarschaft rund um jeden Zellkern berücksichtigt und die Verteilungen der Elliptizität mit einem 2-Komponenten-Gemisch eines Gaußschen(MoG)-Modells modelliert.

10. Verfahren oder System nach Anspruch 6, wobei die Population gegenüber einer erwarteten Anzahl von Zellkernen unter einer kompletten räumlichen Zufälligkeitshypothese verglichen wird.

11. Verfahren oder System nach Anspruch 5, wobei die Anzahl von ternären Merkmalen ein Cribriform-Merkmal, das einen Grad angibt, zu dem die bestimmten der Gewebekomponenten ein Cribriform-Muster aufweisen, und ein Lattenzaun-Merkmal umfasst, das einen Grad angibt, zu dem die bestimmten der Gewebekomponenten ein Lattenzaun-Muster aufweisen.

12. Verfahren nach Anspruch 1 oder System nach Anspruch 2, wobei jedes binäre Merkmal eine gemeinsame Verteilung von z-Scores von den unären Merkmalen davon mit einem Zweikomponenten-, Zweidimensionen-Gemischs einer Gaußschen Verteilung umfasst.

13. Verfahren nach Anspruch 11, wobei die Anzahl von unären Merkmalen das Zellkernkleinheitsmerkmal, das Zellkerngrößenmerkmal, das Zellkernrundheitsmerkmal, das Zellkernelliptizitätsmerkmal, das Zellkernballungsmerkmal und das Zellkernbeabstandungsmerkmal umfasst, wobei die Anzahl von binären Merkmalen ein Zellkernrundheitsmerkmal, ein Zellkernkleinheits-Elliptizitätsmerkmal, ein Zellkernbeabstandungs-Größenmerkmal, ein Zellkernballungs-Kleinheitsmerkmal, ein Zellkernbeabstandungs-Kleinheitsmerkmal, ein Zellkernballungs-Elliptizitätsmerkmal und ein Zellkernbeabstandungs-Rundheitsmerkmal umfasst, und wobei die Anzahl von ternären Merkmalen ein Zellkerngrößen-Rundheits-Beabstandungsmerkmal, das Cribiform-Merkmal und das Lattenzaun-Merkmal umfasst.

14. Verfahren nach Anspruch 1 oder System nach Anspruch 2, wobei die Anzahl von Erkrankungsgebilden eine Anzahl von organspezifischen Erkrankungsgebilden, einschließlich einer Tumor- sowie Nicht-Tumorpathologie, umfasst.

15. Nichtflüchtiges computerlesbares Medium, das ein oder mehrere Programme, einschließlich Befehlen, speichert, die, wenn sie durch einen Computer ausgeführt werden, bewirken, dass der Computer ein Verfahren nach Anspruch 1 durchführt.

## Revendications

1. Procédé de pathologie mis en œuvre par ordinateur, comprenant les étapes consistant à :
recevoir des données d'imagerie cellulaire et/ou sous-cellulaire à plusieurs paramètres pour une image d'un échantillon de tissu ;
localiser et segmenter une pluralité de composants de tissus de l'échantillon de tissu dans les données d'imagerie cellulaire et sous-cellulaire à plusieurs paramètres pour générer des données d'imagerie cellulaire et sous-cellulaire à plusieurs paramètres segmentées ; et
appliquer un schéma de modélisation paramètrique de caractéristiques à certain des composants de tissu dans les données d'imagerie cellulaire et sous-cellulaire à plusieurs paramètres segmentées, dans lequel le schéma de modélisation paramètrique de caractéristiques est généré à partir d'un dictionnaire de motifs histologiques pré-existants pertinents pour le diagnostic et comprend un certain nombre de caractéristiques structurelles adaptées pour définir un certain nombre d'entités de maladie d'une maladie, dans lequel le nombre de caractéristiques structurelles comprend un certain nombre de caractéristiques unaires, dans lesquelles chacune des caractéristiques unaires est une caractéristique morphologique unique, un certain nombre de caractéristiques binaires dans lesquelles chacune des caractéristiques binaires est une combinaison par paire de deux des caractéristiques unaires, et un certain nombre de caractéristiques ternaires dans lesquelles chaque caractéristique ternaire est une combinaison de trois caractéristiques ou plus sélectionnées parmi les caractéristiques unaires ou d'autres caractéristiques structurelles, et dans lequel l'application comprend la détermination d'une quantification de chacune des caractéristiques structurelles pour l'échantillon de tissu ; et
classer un état de la maladie dans l'échantillon de tissu sur la base de la quantification déterminée de chacune des caractéristiques structurelles.

2. Système de pathologie informatique informatisé pour discriminer des motifs de tissu de diagnostic dans des données d'imagerie cellulaire et sous-cellulaire à plusieurs paramètres pour un certain nombre d'échantillons de tissu provenant d'un certain nombre de patients ou d'un certain nombre de modèles in vitro multicellulaires, comprenant :
un appareil de traitement, dans lequel l'appareil de traitement inclut un certain nombre de composants configurés pour :
localiser et segmenter une pluralité de composants de tissu de l'échantillon de tissu dans les données d'imagerie cellulaire et sous-cellulaire à plusieurs paramètres pour générer des données d'imagerie cellulaire et sous-cellulaire à plusieurs paramètres segmentées ;
appliquer un schéma de modélisation paramètrique de caractéristiques à certain des composants de tissu dans les données d'imagerie cellulaire et sous-cellulaire à plusieurs paramètres segmentées, dans lequel le schéma de modélisation paramètrique de caractéristiques est généré à partir d'un dictionnaire de motifs histologiques pré-existants pertinents pour le diagnostic et comprend un certain nombre de caractéristiques structurelles adaptées pour définir un certain nombre d'entités de maladie d'une maladie, dans lequel le nombre de caractéristiques structurelles comprend un certain nombre de caractéristiques unaires, dans lesquelles chacune des caractéristiques unaires est une caractéristique morphologique unique, un certain nombre de caractéristiques binaires dans lesquelles chacune des caractéristiques binaires est une combinaison par paire de deux des caractéristiques unaires, et un certain nombre de caractéristiques ternaires dans lesquelles chaque caractéristique ternaire est une combinaison de trois caractéristiques ou plus sélectionnées parmi les caractéristiques unaires ou d'autres caractéristiques structurelles, et dans lequel l'application comprend la détermination d'une quantification de chacune des caractéristiques structurelles pour l'échantillon de tissu ; et
classer un état de la maladie dans l'échantillon de tissu sur la base de la quantification déterminée de chacune des caractéristiques structurelles.

3. Procédé selon la revendication 1 ou système selon la revendication 2, dans lequel les caractéristiques structurelles incluent un certain nombre de caractéristiques de morphologie cellulaire et un certain nombre de caractéristiques d'organisation cellulaire spatiale.

4. Procédé ou système selon la revendication 3, dans lequel la localisation et la segmentation de la pluralité de composants de tissu comprennent la localisation et la segmentation d'une pluralité de noyaux dans l'échantillon de tissu, les certains des composants de tissu comprenant la pluralité de noyaux, et dans lequel le nombre de caractéristiques de morphologie cellulaire inclut un certain nombre de caractéristiques de taille basées chacune sur une taille nucléaire de chacun des noyaux, un certain nombre de caractéristiques de forme basées chacune sur une forme nucléaire de chacun des noyaux, et un certain nombre de caractéristiques d'étalement spatial basées chacune sur un degré d'espacement nucléaire de chacun des noyaux.

5. Procédé ou système selon la revendication 4, dans lequel le nombre de caractéristiques de taille inclut une caractéristique de petitesse nucléaire et une caractéristique de largeur nucléaire, dans lequel le nombre de caractéristiques de forme comprend une caractéristique de rondeur nucléaire et une caractéristique d'ellipticité nucléaire, et dans lequel le nombre de caractéristiques d'étalement spatial comprend une caractéristique d'encombrement nucléaire et une caractéristique d'espacement nucléaire.

6. Procédé ou système selon la revendication 5, dans lequel :
(i) la caractéristique de petitesse nucléaire est basée sur un premier histogramme de zones nucléaires obtenues à partir de régions prototypiques contenant des noyaux d'une première classification de taille comprenant une petite classification, dans lequel le premier histogramme est modélisé avec une distribution Gamma, et dans lequel la caractéristique de largeur nucléaire est basée sur un second histogramme de zones nucléaires obtenues à partir de régions prototypiques contenant des noyaux d'une seconde classification de taille comprenant une grande classification, dans lequel le second histogramme est modélisé avec une distribution Gamma ; ou
(ii) la caractéristique d'encombrement nucléaire est quantifiée en calculant, pour chaque noyau, une distance moyenne par rapport à une pluralité de noyaux voisins les plus proches ; ou
(iii) la caractéristique d'espacement nucléaire est quantifiée en plaçant, pour chaque noyau, une cellule de grille centrée au niveau d'un noyau de référence et en mesurant une densité d'une pluralité de noyaux voisins en comptant une population de noyaux dans la cellule de grille.

7. Procédé ou système selon la revendication 5, dans lequel la caractéristique de rondeur nucléaire est basée sur un certain nombre de premières mesures données chacune par (4*π* x aire)/périmètre" et dans lequel la caractéristique d'ellipticité nucléaire est basée sur un nombre de secondes mesures données chacune à partir d'un rapport d'une longueur d'un axe mineur à une longueur d'un axe majeur.

8. Procédé ou système selon la revendication 7, dans lequel la caractéristique de rondeur nucléaire est comprise entre 0, ce qui indique une apparence irrégulière en forme d'étoile, et 1, ce qui indique un cercle parfait, et dans lequel la caractéristique d'ellipticité nucléaire caractérise une planéité d'un noyau, des valeurs inférieures indiquent des noyaux hautement elliptiques.

9. Procédé selon la revendication 7, dans lequel la caractéristique de rondeur nucléaire considère un voisinage spatial autour de chaque noyau et modélise les distributions de rondeur avec une distribution Gamma, et dans lequel la caractéristique d'ellipticité nucléaire considère un voisinage spatial autour de chaque noyau et modélise les distributions d'ellipticité avec un mélange à 2 composants de modèle gaussien (MoG).

10. Procédé ou système selon la revendication 6, dans lequel la population est comparée à un certain nombre attendu de noyaux dans une hypothèse de hasard spatial complet.

11. Procédé ou système selon la revendication 5, dans lequel le nombre de caractéristiques ternaires inclut une caractéristique cribriforme indicative d'un degré auquel certains des composants de tissu présentent un motif cribriforme et une caractéristique de piquet de clôture indicative d'un degré auquel certains des composants de tissu présentent un motif de piquet de clôture.

12. Procédé selon la revendication 1 ou système selon la revendication 2, dans lequel chaque caractéristique binaire comprend une distribution conjointe de scores z à partir de ses caractéristiques unaires avec un mélange bidimensionnel à deux composants de distribution gaussienne.

13. Procédé ou système selon la revendication 11, dans lequel le nombre de caractéristiques unaires comprend la caractéristique de petitesse nucléaire, la caractéristique de grande taille nucléaire, la caractéristique de rondeur nucléaire, la caractéristique d'ellipticité nucléaire, la caractéristique de densité nucléaire et la caractéristique d'espacement nucléaire, dans lequel le nombre de caractéristiques binaires inclut une caractéristique de grande taille-rondeur nucléaire, une caractéristique de petite taille-ellipticité nucléaire, une caractéristique d'espacement-grande taille nucléaire, une caractéristique d'encombrement-petite taille nucléaire, une caractéristique d'espacement-petite taille nucléaire, une caractéristique d'encombrement-ellipticité nucléaire et une caractéristique d'espacement-rondeur nucléaire, et dans lequel le nombre de caractéristiques ternaires inclut une caractéristique de grande taille-rondeur-espacement nucléaire, la caractéristique cribriforme et la caractéristique de piquet de clôture.

14. Procédé selon la revendication 1 ou système selon la revendication 2, dans lequel le nombre d'entités de maladie comprend un nombre d'entités de maladie spécifiques à un organe, incluant à la fois une pathologie tumorale et non tumorale.

15. Support non transitoire lisible par ordinateur stockant un ou plusieurs programmes, incluant des instructions qui, lorsqu'elles sont exécutées par un ordinateur, amène l'ordinateur à exécuter le procédé selon la revendication 1.
